Europäisches Patentamt

European Patent Office

Office européen des brevets

(11) Veröffentlichungsnummer: **0 299 303 B1**

(12) **EUROPÄISCHE PATENTSCHRIFT**

(45) Veröffentlichungstag der Patentschrift: **03.03.93**

(21) Anmeldenummer: **88110593.6**

(22) Anmeldetag: **02.07.88**

(51) Int. Cl.5: **C12N 15/85**, C12N 15/67, C12N 15/64

(54) **Eukaryotische Expressionsvektoren mit multimeren Enhancer-Subelementen, Verfahren zu ihrer Herstellung und Verwendung.**

(30) Priorität: **11.07.87 DE 3723075**

(43) Veröffentlichungstag der Anmeldung:
**18.01.89 Patentblatt 89/03**

(45) Bekanntmachung des Hinweises auf die
Patenterteilung:
**03.03.93 Patentblatt 93/09**

(84) Benannte Vertragsstaaten:
**AT BE CH DE ES FR GB GR IT LI LU NL SE**

(56) Entgegenhaltungen:
**WO-A-85/05629**

**MOLECULAR AND CELLULAR BIOLOGY,
Band 3, Nr. 7, Juli 1983, Seiten 1246-1254,
American Society for Microbiology, US; P.E.
BERG et al.: "Differential activation of the
mouse beta-globin promoter by enhancers"**

(73) Patentinhaber: **BOEHRINGER MANNHEIM
GMBH
Sandhofer Strasse 116
W-6800 Mannheim 31(DE)**

(72) Erfinder: **Schaffner, Walter, Prof. Dr.
Bodenstrasse 9
CH-8104 Weiningen(CH)**
Erfinder: **Westin, Nils Gunnar, Dr.
Norrtäljegatan 5A
S-753 27 Uppsala(SE)**

PROCEEDINGS OF A CETUS-UCLA SYMPOSI-
UM, GENE EXPRESSION, Park City, Utah, 26.
März - 1. April 1983, Seiten 107-124, herausgegeben von D.H. Hamer et al., A.R. LISS,
Inc., New York, US; M. KRIEGLER et al.:
"Promoter substitution and enhancer augmentation increases the penetrance of the
SV40 a gene to levels comparable to that of
the harvey murine sarcoma virus ras gene in
morphologic transformation"

NUCLEIC ACIDS RESEARCH, Band 15, Nr. 17,
1987, Seiten 6787-6798; G. WESTIN et al.:
"OVEC, a versatile system to study transcription in mammalian cells and cell-free
extracts"

THE EMBO JOURNAL, Band 7, Nr. 12, 1988,
Seiten 3763-3770; G. WESTIN et al.: "A zincresponsive factor interacts with a metalregulated enhancer element (MRE) of the
mouse metallothionein-I gene"

MOLECULAR AND CELLULAR BIOLOGY,
Band 5, Nr. 4, April 1985, Seiten 649-658,
American Society for Microbiology; G.M.
VELDMAN et al.: "Polyomavirus enhancer
contains multiple redundant sequence elements that activate both DNA replication
and gene expression"

NUCLEIC ACIDS RESEARCH, Band 12, Nr. 16,
1984, Seiten 6427-6442; L.A. LAIMINS et al.:
"Multiple enhancer domains in the 3' terminus of the Prague strain of Rous sarcoma
virus"

**Beschreibung**

Die Erfindung betrifft eukaryotische Expressionsvektoren mit multimeren Enhancer-Elementen, Verfahren zu ihrer Herstellung und ihre Verwendung zur Expression von Proteinen in Säugerzellen.

Die Expression von Proteinen (im folgenden als heterologe Proteine bezeichnet) in Säugerzellen ist aus therapeutischer und diagnostischer Sicht von besonderem Interesse.

Die Expression von heterologen Proteinen in Säugerzellen ist bisher nur für wenige Zellinien (z. B. CHO-Zellen, DNA 3 (1984) 297 - 308, Mol. and Cell. Biol. 5 (1985) 1750 - 1759 und Mol und Cell. Biol. 4 (1984) 166 - 172, Adeno-Vektor in Hela-Zellen, [PNAS 82 (1985), 3567 - 3571]) bekannt und verläuft dabei, im Vergleich zur Expression von Fremdgenen in Prokaryonten, nur mit schwacher Ausbeute.

Üblicherweise werden in Säugerzellen Vektoren mit Elementen von Viren verwendet. Beispiele hierfür sind Bovine papilloma virus [PNAS 81 (1984), 5086 - 5090], Simian virus 40 [PNAS 82 (1985), 3644 - 3648], retroviral elements [DNA 4 (1985), 23 - 31] oder adenovirus elements [PNAS 82 (1985), 3567 - 3571].

Solche Vektoren basieren meist auf eukaryotischen Virus-DNA- Strukturen und enthalten im wesentlichen das Gen des zu exprimierenden Proteins (heterologes Gen), Resistenzgene für die Selektion, einen Promotor für das heterologe Gen, einen Ursprungspunkt der DNA-Replikation (ori) sowie aktivierende DNA-Sequenzen (Enhancer-Elemente) (vgl. Science 209 (1980), 1422 - 1427). Für die Präparation von Vektor-DNA in Bakterien kann der Vektor in einem bakteriellen Plasmid kloniert sein. Die Enhancer-Elemente sind meist oberhalb (5$'$) des zu exprimierenden Gens, entweder außerhalb oder übergehend in die Promotor-Sequenz, angeordnet. Enhancer-Elemente entfalten ihre Aktivität auch in einer Position innerhalb oder unterhalb (3$'$) der Transkriptionseinheit. Die Länge dieser Sequenzen beträgt meist zwischen 70 und 300 bp (SV40 Enhancer, Polyomavirus Enhancer, Nucl. Acids Res. 10 (1982), 7965 - 7976), aber es wurden auch schon wesentlich längere Enhancer-Elemente gefunden (Cell 41 (1985), 521 - 530, PNAS 82 (1985) p. 8325 - 8329). Die Enhancer-Elemente können die eukaryotische Transkription stimulieren und sind deshalb von besonderem Interesse. Diese stimulierende Wirkung ist weitgehend unabhängig von der Lage und Orientierung des Enhancer Elements im Vektor (Cell. 27 (1981), 299 - 308).

Enhancer-Elemente werden in eukaryotischen Viren (z. B. SV40, Polyomavirus, BPV, Cytomegalovirus, MSV, HIV) sowie in eukaryotischen Genen, wie z. B. den Genen für Immunglobulin, Interferon, Chymotrypsin und Insulin, gefunden. Bei SV40 ist die Enhancer-Funktion größtenteils in einem 72 bp langen Repeat, welcher sich flußaufwärts vom Transkriptionsstart befindet, lokalisiert, Cell. 27 (1981), 299 - 308). In diesem 72 bp Repeat ist eine core-Sequenz enthalten, die wesentlich zur Enhancer-Funktion beiträgt (Science 219 - (1983), 626 - 631). Eine solche core-Sequenz is jedoch allein kein brauchbarer Enhancer (Genes Dev. 1 - (1987) 65 - 73). Sie muß in beträchtlichem Umfang von ihren natürlichen Nachbarsequenzen umgeben sein (Cell. 39 (1984), 653 - 662). Aber auch mit derartigen Enhancer-Elementen konnte bisher nur eine Aktivität von maximal 5 % des Wildtyps erreicht werden (Mol. and Cell. Biol. 5 (1985), 649 - 658). Eine wesentliche Erhöhung der Expression findet statt, wenn die Enhancer-Elemente multimerisiert werden. So konnte für den Polyomavirus-Enhancer mit einem Segment von 26 Nucleotiden, welches in 5 - 7 Kopien im Vektor enthalten war, sogar eine geringfügig höhere Transkriptionsrate als beim Wildtyp beobachtet werden (Mol. and Cell. Biol. 5 (1985), 649 - 658).

Im Vergleich zur Expression von Fremdgenen in Prokaryonten, verläuft die Expression in Eukaryonten, unter Verwendung der bekannten Promotoren, jedoch nur mit einer vergleichsweise geringen Ausbeute.

Aufgabe der vorliegenden Erfindung ist daher ein verbessertes Expressionssystem zu schaffen, welches die Expression von Fremdgenen in Säugerzellen mit hoher Ausbeute gewährleistet.

Diese Aufgabe wird durch einen eukaryotischen Expressionsvektor, der ein zu exprimierendes Gen und eine TATA-Box enthält, gelöst, der dadurch gekennzeichnet ist, daß 1 - 200 Basenpaare (bp) oberhalb (5$'$) der TATA-Box ein erstes multimeres Enhancer-Subelement und 1 - 1000 bp unterhalb (3$'$) des zu exprimierenden Gens ein zweites multimeres Enhancer-Subelement angeordnet ist.

Überraschenderweise hat sich gezeigt, daß mit den erfindungsgemäßen Expressionsvektoren eine beträchtlich höhere Expression erreicht werden kann, als mit allen bisher bekannten Systemen. Dies ist um so überraschender, da mit Vektoren, welche nur das erste oder nur das zweite multimere Enhancer-Subelement enthalten, keine besonders hohe Expression beobachtet werden konnte.

Die multimeren Enhancer-Subelemente bestehen aus 3 - 30 hintereinandergeschalteten einzelnen Enhancer-Subelementen. Verzugsweise ist das erste multimere Subelement ein 3 - 10-meres und das zweite multimere Subelement ein 5 - 30-meres. Besonders bevorzugt werden Multimere als Tandemkopien ("direct repeats") von Subelementen verwendet. Das 3$'$Ende des ersten Elements ist 1 - 200 Basenpaare oberhalb (5$'$) der TATA-Box, vorzugsweise 8 - 20 Basenpaare oberhalb der TATA-Box angeordnet. Das 5$'$-Ende des zweiten multimeren Enhancer-Subelementes ist 1 - 1000 bp, vorzugsweise 10 - 300 bp unterhalb (3$'$) des zu exprimierenden Gens angeordnet.

Die einzelnen Enhancer-Subelemente haben eine Länge von 15 - 60 bp und bestehen aus mindestens einem, vorzugsweise 1 - 7 Enhancer-Sequenzmotiven, können jedoch neben den Sequenzmotiven auch flankierende Nachbarsequenzen enthalten. Die Nachbarsequenzen können den in natürlichen Enhancern vorkommenden Nachbarsequenzen der Sequenzmotive entsprechen, aber auch beliebige andere Sequenzen sein.

Unter Enhancer-Sequenzmotiven sind diejenigen Sequenzen eines Enhancers zu verstehen, die für die Enhancer-Wirkung essentiell sind und demzufolge als der funktionell wirksame Bereich des Enhancers bezeichnet werden. Die Länge dieser Enhancer-Sequenzmotive beträgt üblicherweise zwischen 8 und 18 bp. Diese Sequenzmotive zeigen häufig eine Homologie mit den Enhancern aus anderen Viren und zellulären Genen.

Beispiele für bevorzugte Enhancer-Sequenzmotive sind (N: beliebiges Nuckleotid):

$^A_C$ GGAAGTG$^A_C$ (Ela core enhancer, Cell 33, 695 - 703 (1983))

GTGG$^A_T$ $^A_T$ $^A_T$ G (Sequenz homolog zu Immunoglobulin und viralen Enhancern, Science 219, 626 - 631 (1983)

TGTGGTAAG (MSV, PNAS 79 (1982) 6453)

GTGTGGAAAG (Science 219 (1983) 626 - 631)

TGGTTG (BPV, Cell 18 (1979) 963)

GTGTGGTTT (Py(F101), Nucleic Acids Res. 9 (1981) 6251.

ATGCAAATNA (Nature 310 (1986), 71 - 74)

TNATTTGCAT (IgH enhancer, Banerji et al., Cell 33 (1983) 729 - 740)

TCAAGATGGC, AGCAGCTGGC, GTCATGTGGC, ACCACCGGGT (Ephrussi-Motive des Maus, IgH-Enhancers, Science 227 (1983) 134 - 140).

Besonders vorteilhaft ist es, Enhancer-Sequenzmotive von humanen, zellulären Genen (z. B. des menschlichen IgH-enhancers, Nature 306, 806 - 809 (1983) zu verwenden. Damit kann die Zellkultur frei von viralen Elementen gehalten werden.

Ein besonders geeignetes Enhancer-Sequenzmotiv ist das MREwt oligonucleotid des Metallothionein-Enhancers:

```
5'-GAGCTCTGCACTCCGCCC
        AGACGTGAGGCGGGCTCG-5'
```

Acht Tandem-Kopien dieses Enhancer-Sequenzmotivs können beispielsweise aus dem 4 x MREwt/8 x MREwt Plasmid (DSM 4098P) mit EcoRI oder XhoI ausgeschnitten werden.

Ein weiterer Gegenstand der Erfindung ist ein Verfahren zur Herstellung eines erfindungsgemäßen Expressionsvektors, welches dadurch gekennzeichnet ist, daß nach bekannten gentechnologischen Standardverfahren ein erstes multimeres Enhancer-Subelement 1 - 200 bp, vorzugsweise 8 - 20 bp oberhalb (5′) der TATA-box und ein zweites multimeres Enhancer-Subelement 1 - 1000 bp, vorzugsweise 10 - 300 bp unterhalb (3′) des zu exprimierenden Gens eingebaut wird. Als Vektoren kommen dabei die üblicherweise für Säugerzellen verwendeten Vektoren, beispielsweise die weiter oben genannten Vektoren in Frage.

Sofern die polymerisierten Enhancer-Oligonukleotide geeignete terminale Sequenzen haben, können sie direkt in dem Vektor ligiert werden. In den anderen Fällen müssen zunächst kompatible Enden für die Ligierung erzeugt werden. Dies geschieht bevorzugt durch Auffüllen von verstehenden Enden mit Klenow Polymerase oder T4 Polymerase. Dabei entstehen flache Enden (blunt ends) für die Ligation.

Ein weiterer Gegenstand der Erfindung ist die Verwendung eines erfindungsgemäßen Expressionsvektors zur Expression von Proteinen in Säugerzellen. Als Säugerzellen geeignet sind solche Zellen, in denen die erfindungsgemäßen Vektoren aktiv sind. Beispiele hierfür sind Vektoren auf SV40-Basis in CHO-Zellen oder Vektoren auf Basis des Plasmids pβ1E (DSM 4097P) in Hela-Zellen.

Die gentechnologischen Standardverfahren, wie Klonierung, DNA-Isolierung, Restriktion werden, wenn nichts anderes angegeben, analog T. Maniatis et al., Molecular Cloning (1982) Cold Spring Harbor Laboratory, CHS, New York, 11724, durchgeführt. Molekularbiologische Reagenzien werden nach Vorschrift der Hersteller eingesetzt.

Die nachfolgenden Beispiele und Abbildungen erläutern die Erfindung weiter:

Fig. 1    Plasmid 4 x MREwt/8 x MREwt

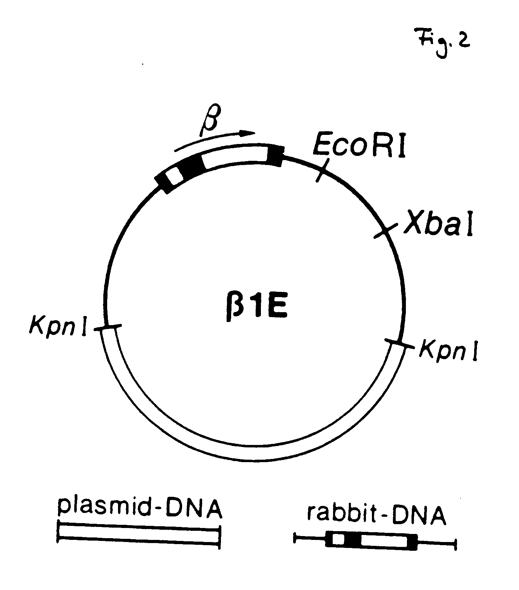

Fig. 2    Plasmid β1E

Beispiel 1

Zwei komplementäre Oligonucleotide (MREwt) der Sequenzen

$$5'-\texttt{GAGCTCTGCACTCCGCCC}-3'$$

$$3'-\texttt{AGACGTGAGGCGGGCTCG}-5'$$

enthaltend ein konserviertes MRE-Enhancer-Sequenzmotiv und eine Spl-Bindungsstelle, werden in äquimolarer Menge vermischt und als Doppelstrang-DNA, unter Verwendung von $T_4$-Ligase, multimerisiert.

Eine Einfügung von Linkern erübrigt sich, da die Doppelstrang-Oligonucleotide komplementäre überhängende Enden besitzen. Nach der Ligation werden die Oligomeren mit $\alpha$-$^{32}$P dNTPs markiert und die noch überhängenden Enden mit T4 DNA Polymerase glatt gemacht.

Die Reaktionsprodukte werden anschließend auf einem nativen Polyacrylamidgel größenfraktioniert, wobei als Standard ein markierter DNA-Molekulargewichtsmarker mitgeführt wird.

Die über Autoradiographie identifizierten 4- und 8-Tandem-Kopien des Oligonucleotids werden aus dem Gel herausgeschnitten und eluiert. Das Tetramere und das Oktamere werden dann, wie nachstehend beschrieben, in das Plasmid p$\beta$1E (DSM 4097P) vor bzw. nach dem Kaninchen $\beta$-Globin-Gen insertiert.

Mit diesen Oligonucleotiden werden folgende Plasmide hergestellt:

a) Plasmid 4 x MREwt (4 Tandem-Kopien oberhalb (5$'$) vom Kaninchen $\beta$-Globingen).

Das Tetramer wird in die glattgemachte Hind III Stelle in den Vektor p$\beta$1E in Position -37 des Kaninchen $\beta$-Globin-Gens eingesetzt. Dieses Plasmid enthält außer der TATA-Box keine weiteren $\beta$-Globingen-Promotorelemente. Außer dem Globin-Gen sind mehrere Kilobasenpaare von dem Globin-Gen downstream benachbarten Sequenzen enthalten.

b) Plasmid 8 x MREwt (8 Tandem-Kopien downstream vom $\beta$-Globin-Gen

Das Oktamer wird zunächst in die Eco RV-site des Vektors pUC 7/X (DSM 4177P ) (welcher den Multilinker Eco RI, Xho I, Eco RV, Xho I, Eco RI in pUC7 integriert enthält) einligiert. Anschließend wird mit Eco RI behandelt und das kleinere Fragment (200 bp) durch präparative Agarosegel-Elektrophorese isoliert und in die Eco RI-site downstream ( + 2 kbp Abstand zum 5$'$-Ende bzw. 700 bp Abstand zum 3$'$-Ende des $\beta$-Globin-Gens) des $\beta$-Globin-Gens im Plasmid p$\beta$1E eingesetzt.

c) Plasmid 4 x MREwt/8 x MREwt (DSM 4098P, dieses Plasmid enthält 4 TandemKopien upstream und 8 Tandem-Kopien downstream von $\beta$-Globin-Gen).

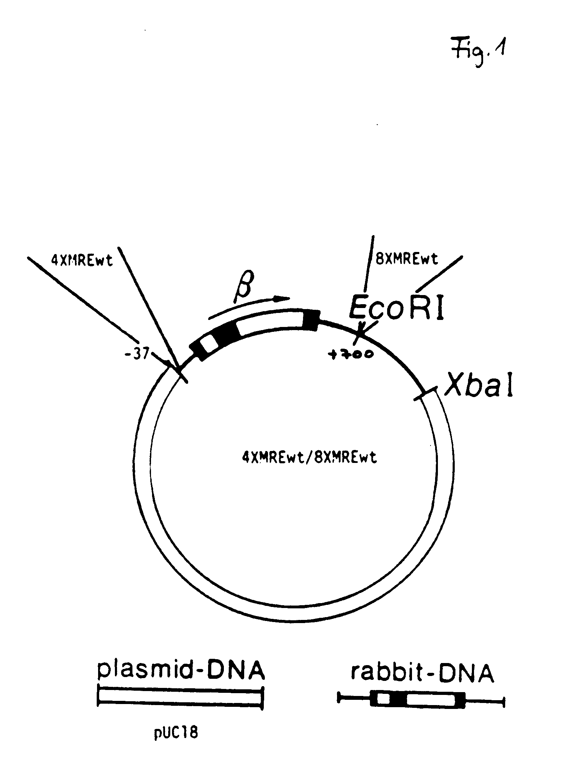

Das 0,5 kbp Eco RI/BamH I-Fragment aus dem Plasmid 4 x MREwt und das 2,1 kbp BamH I/Xba I-Fragment aus dem Plasmid 8 x MREwt werden in die Eco RI/Xba I-site des Vektors M13mp18 bzw. pUC 18 (Sequenz: Gene 33 (1985) 103 - 119) einligiert (Fig. 1).

Die Plasmide werden in E.coli HB 101 (DSM 1607) kloniert und anschließend isoliert.

Beispiel 2

Plasmid SV40 enhancer (Plasmid, welches ein SV40 Enhancer-Element unterhalb (3$'$) vom $\beta$-Globin-Gen enthält).

Ein 200 bp-Fragment von SV40 (Weber et al., Cell. 36 (1984), 983 - 995), welches den Nucleotiden 95 - 294 von SV40 entspricht, wird auf der 5$'$-Seite mit einem XhoI/Eco RI und auf der 3$'$-Seite mit einem Xho I-Linker versehen. Die Enden werden glattgemacht und direkt in die Eco RI-site von Plasmid p$\beta$1E eingesetzt.

Beispiel 3

Transfektion von eukaryotischen Zellen mit den Plasmiden aus Beispiel 1 und 2.

Hela-Zellen (ATCC Nr. CCL2, adhärente Zellen) werden nach der Calciumphosphat-Transfektions-Methode, wie sie beispielsweise in Wigler et al., Cell 14 (1978), 725 - 731 und in Weber et al., Cell 36 - (1984), 983 - 992 beschrieben ist, mit den Plasmiden aus den Beispielen 1 und 2 transfektiert. Zur Bestimmung der Transkriptionsaktivität der Zellen wird das Medium abgesaugt, 10 ml TBS (25 mmol/l Tris-HCl pH 7,4, 137 mmol/l NaCl, 5 mmol/l kCl, 0.6 mmol/l $Na_2HPO_4$) zugegeben und nach wenigen Minuten Inkubationszeit bei Raumtemperatur nochmals abgesaugt. Anschließend werden 4 ml 25 %iges Dimethylsulfoxid (DMSO) in TBS zugegeben und 2 - 5 min bei Raumtemperatur inkubiert. Anschließend wird abgesaugt, 10 ml TBS zugegeben, abgesaugt und nochmals mit TBS gewaschen. Anschließend wird frisches Dulbecco's modified Eagle Medium zugegeben.

Eine Stunde nach dem DMSO-Schock werden 0,1 mmol/l Zinkionen und 0,5 µMol/l Cadmiumionen zur MRE-Induktion zugegeben. Nach einer Inkubationszeit von 6 - 8 Stunden bei 37° werden Metallionen bis zu einer Endkonzentration von 0,2 mmol/l Zink und 1 µMol/l Cadmium zugegeben.

Zur Bestimmung der Transkriptionsaktivität wird die cytoplasmatische RNA 40 - 46 Stunden nach der Transfektion isoliert und durch SI-Nuclease-Analyse und Polyacrylamid-Gel-Elektrophorese bestimmt (Techniques in the Life Sciences, (B5) Nucleic Acid Biochemistry, Ed. Flavell (1983), R. A. Elsevier Scientific Publishers Ireland, Ltd., S. 1 - 20). Tabelle I zeigt die Ergebnisse. Daraus ist zu ersehen, daß mit dem Plasmid gem. Beispiel 1c eine im Vergleich zu den bekannten aktivsten Enhancer-Elementen von SV40 eine um das 20 - 30fach höhere Transkriptionsaktivität erhalten werden kann.

## Tabelle I

### Transkriptionsaktivität für Hela-Zellen nach Transfektion mit verschiedenen Plasmiden

| Plasmid | relative Aktivität |
|---|---|
| 4 x MREwt (Bsp. 1a) | 3 |
| 8 x MREwt (Bsp. 1b) | 3 |
| 4 x MREwt/8 x MREwt (Bsp. 1c) | 20 - 30 |
| pß1E/SV40 (Bsp. 2) | 1 |

(Bestimmung der Aktivität durch visuelle Beurteilung der Röntgenfilme.)

**Patentansprüche**

1. Eukaryotischer Expressionsvektor, enthaltend ein zu exprimierendes Gen und eine TATA-box, dadurch gekennzeichnet, daß 1 - 200 Basenpaare (bp) oberhalb (5′) der TATA-box ein erstes multimeres Enhancer-Subelement und 1 - 1000 bp unterhalb (3′) des zu exprimierenden Gens ein zweites multimeres Enhancer-Subelement angeordnet ist.

2. Eukaryotischer Expressionsvektor nach Anspruch 1, dadurch gekennzeichnet, daß 8 - 20 bp oberhalb (5′) der TATA-box ein erstes multimeres Enhancer-Subelement und 10 - 300 bp unterhalb (3′) des zu exprimierenden Gens ein zweites multimeres Enhancer-Subelement angeordnet ist.

3. Eukaryotischer Expressionsvektor nach den Ansprüchen 1 und 2 dadurch gekennzeichnet, daß die multimeren Enhancer-Subelemente eine Länge von 15 - 60 bp haben.

4. Eukaryotischer Expressionsvektor nach den Ansprüchen 1 - 3, dadurch gekennzeichnet, daß die multimeren Enhancer-Subelemente 3 - 30-mer sind.

5. Verfahren zur Herstellung eines eukaryotischen Expressionsvektors gem. den Ansprüchen 1 - 4, dadurch gekennzeichnet, daß nach bekannten Methoden ein erstes multimeres Enhancer-Subelement 1 - 200 bp oberhalb (5′) der TATA-box und ein zweites multimeres Enhancer-Subelement 1 - 1000 bp unterhalb (3′) des zu exprimierenden Gens eingebaut wird.

6. Verwendung eines eukaryotischen Expressionsvektors nach den Ansprüchen 1 - 4 zur Expression von Proteinen in Säugerzellen.

## Claims

1. Eukaryotic expression vector containing a gene to be expressed and a TATA box, characterised in that 1 - 200 base pairs (bp) above (5') of the TATA box there is provided a first multimeric enhancer subelement and 1 - 1000 bp below (3') of the gene to be expressed there is provided a second multimeric enhancer element.

2. Eukaryotic expression vector according to claim 1, characterised in that 8 - 20 bp above (5') of the TATA box there is provided a first multimeric enhancer subelement and 10 - 300 bp below (3') of the gene to be expressed there is provided a second multimeric enhancer subelement.

3. Eukaryotic expression vector according to claims 1 and 2, characterised in that the multimeric enhancer subelements have a length of 15 - 60 bp.

4. Eukaryotic expression vector according to claims 1 - 3, characterised in that the multimeric enhancer subelements are 3 - 30 mers.

5. Process for the production of a eukaryotic expression vector according to claims 1 - 4, characterised in that, according to known methods, a first multimeric enhancer subelement is incorporated 1 - 200 bp above (5') of the TATA box and a second multimeric enhancer subelement is incorporated 1 - 1000 bp below (3') of the gene to be expressed.

6. Use of a eukaryotic expression vector according to claims 1 - 4 for the expression of proteins in mammalian cells.

## Revendications

1. Vecteur d'expression eucaryotique, contenant un gène à exprimer et une boîte TATA, caractérisé en ce que, à 1-200 paires de bases (bp) au-dessus de (5') de la boîte TATA, est disposé un premier sous-élément facilitateur multimérique et à 10-300 bp en dessous de (3') du gène à exprimer, est disposé un deuxième sous-élément facilitateur multimérique.

2. Vecteur d'expression eucaryotique selon la revendication 1, caractérisé en ce que, à 8-20 bp au dessus de (5') de la boîte TATA, est disposé un premier sous-élément facilitateur multimérique et à 10-300 bp en dessous de (3') du gène à exprimer, est disposé un deuxième sous-élément facilitateur multimérique.

3. Vecteur d'expression eucaryotique selon les revendications 1 et 2, caractérisé en ce que les sous-éléments facilitateurs multimériques ont une longueur de 15-60 bp.

4. Vecteur d'expression eucaryotique selon l'une quelconque des revendications 1-3, caractérisé en ce que les sous-éléments facilitateurs multimériques sont des 3-30-mères.

5. Procédé pour la préparation d'un vecteur d'expression eucaryotique selon les revendications 1-4, caractérisé en ce que selon des procédés connus, un premier sous-élément facilitateur multimérique est incorporé à 1-200 bp au-dessus de (5') de la boîte TATA et un second sous-élément facilitateur multimérique est incorporé à 1-1000 bp en-dessous de (3') du gène à exprimer.

6. Utilisation d'un vecteur d'expression eucaryotique selon les revendications 1-4, pour l'expression de protéines dans des cellules extractrices.

*Fig. 1*

*Fig. 2*

β1E

β

*Eco*RI

*Xba*I

*Kpn*I

*Kpn*I

plasmid-DNA

rabbit-DNA